(19) Europäisches Patentamt
European Patent Office
Office européen des brevets

(11) Veröffentlichungsnummer : **0 453 525 B1**

# (12) EUROPÄISCHE PATENTSCHRIFT

(45) Veröffentlichungstag der Patentschrift : **28.04.93 Patentblatt 93/17**

(51) Int. Cl.[5] : **A61K 9/107,** A61K 47/28

(21) Anmeldenummer : **90915080.7**

(22) Anmeldetag : **11.10.90**

(86) Internationale Anmeldenummer : **PCT/DE90/00779**

(87) Internationale Veröffentlichungsnummer : **WO 91/07170 30.05.91 Gazette 91/12**

## (54) VERFAHREN ZUR HERSTELLUNG WÄSSRIGER MISCHMICELLÖSUNGEN.

(30) Priorität : **13.11.89 DE 3938030**

(43) Veröffentlichungstag der Anmeldung : **30.10.91 Patentblatt 91/44**

(45) Bekanntmachung des Hinweises auf die Patenterteilung : **28.04.93 Patentblatt 93/17**

(84) Benannte Vertragsstaaten : **AT BE CH DE DK ES FR GB IT LI LU NL SE**

(56) Entgegenhaltungen :
**EP-A- 0 252 004**
**EP-A- 0 280 887**
**DE-A- 2 730 570**
**FR-A- 1 289 401**

(73) Patentinhaber : **SCHERING AKTIENGESELLSCHAFT Berlin und Bergkamen**
**Müllerstrasse 170/178 Postfach 65 03 11**
**W-1000 Berlin 65 (DE)**

(72) Erfinder : **RÖSSLING, Georg**
**Oranienburger Chaussee 60 C**
**W-1000 Berlin 28 (DE)**
Erfinder : **GÖRITZ, Detlef**
**Im Wolfshorst 45**
**W-1000 Berlin 20 (DE)**
Erfinder : **MICHEL, Heinrich**
**Seeburger Str. 5**
**W-1000 Berlin 20 (DE)**

EP 0 453 525 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung wässriger Mischmicelllösungen, enthaltend aus Lipoiden und Salzen von Gallensäuren gebildete Mischmicellen, in denen gewünschtenfalls in Wasser schwer lösliche oder unlösliche Wirkstoffe solubilisiert sind.

Verfahren zur Herstellung derartiger Mischmicellösungen sind beispielsweise aus der deutschen Patentschrift 27 30 570 vorbekannt.

Bei den vorbekannten Verfahren werden die Mischmicellösungen hergestellt, indem man die Lipoide, die Salze der Gallensäuren und gegebenenfalls die in Wasser schwer löslichen oder urilöslichen Wirkstoffe in einem organischen Lösungsmittel (z.B. Ethanol) löst, und die Lösungen einengt, so daß sich an den Gefäßwänden ein Lipidfilm bildet, der mittels wässrigen Lösungen abgelöst wird. (Biochemistry 19, 1980, 602 ff und 615 ff; Naturforsch. 32c, 1977, 748 ff).

Dieses Verfahren ist aber recht aufwendig und nur mit einem erheblichen apparativen Aufwand in einen technisch nutzbaren Maßstab zu übertragen.

Neben diesem bevorzugten Verfahren ist beispielsweise aus Beispiel 3 der bereits erwähnten Patentschrift 27 30 570 ein Verfahren bekannt, bei dem man derartige Mischmicellösungen durch Mischen der Komponenten und Rühren der Mischung herstellt.

Dieses Verfahren hat aber nicht nur den Nachteil, daß es mehrere Tage dauert, sondern man erkennt bei der Nacharbeitung dieses Beispiels - ohne Wirkstoff -daß man auf diese Weise nur stark getrübte Lösungen erhält, die Mischmicellen mit einem mittleren Durchmesser von ca. 340 nm enthalten.

Klare Lösungen mit Mischmicellen von einem mittleren Durchmesser von ca 10 nm sind auf diese Weise nicht zu erhalten.

Es wurde nun gefunden, daß man in einfacher Weise und kurzer Zeit derartig klare wässrige Lösungen von Mischmicellen mittels eines Verfahrens herstellen kann, welches dadurch gekennzeichnet ist, daß man Mischungen aus

a) Lösungen, welche die Lipide, die freien Gallensäuren und gegebenenfalls die in Wasser schwer löslichen oder unlöslichen Wirkstoffe in einem wasserlöslichen organischem Lösungsmittel enthalten und

b) Lösungen die bezogen auf die Gallensäuren 0,05 bis 3 Aquivalente Basen und gegebenenfalls isotonisierende Zusätze und/oder wasserlösliche Wirkstoffe enthalten,

bereitet, das organische Lösungsmittel durch Ultrafiltration, Lyophyliisieren, Vakuumdestillation oder Umkehrosmose entfernt und die erhaltene Mischung gewünschtenfalls mit wässriger Phase verdünnt.

Das erfindungsgemäße Verfahren kann unter Verwendung der gleichen Gallensäuren durchgeführt werden wie die vorbekannten Verfahren. Geeignete Gallensäuren sind 5β-Cholan-24-säure-Derivate der allgemeinen Formel

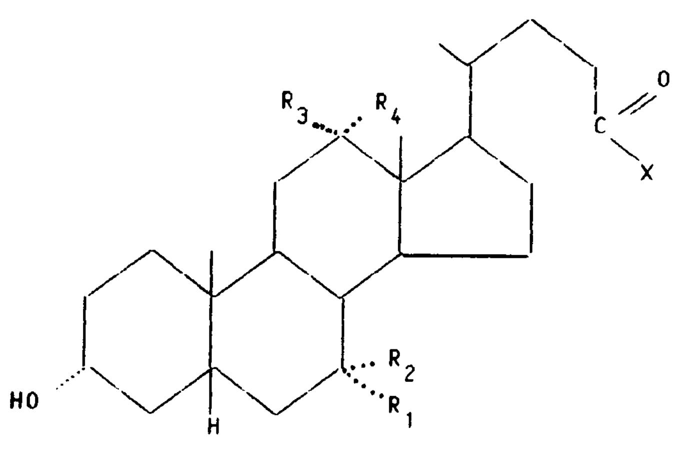

worin

$R_1$ und $R_2$ sowie $R_3$ und $R_4$ gemeinsam eine Oxogruppe, zwei Wasserstoffatome oder ein Wasserstoffatome und eine Hydroxygruppe bedeuten und X eine Hydroxygruppe oder eine Gruppierung der Formel $-NH-CH_2-CO_2H$ oder $-NH-(CH_2)_2-SO_3H$ darstellt.

Als geeignete Gallensäuren seien beispielsweise genannt: Die Cholsäure, die Glycocholsäure, die Taurocholsäure, die Deoxycholsäure, die Glycodeoxycholsäure, die Taurodeoxycholsäure, die Chenodeoxycholsäure, die Glycochenodeoxycholsäure und die Taurochenodeoxycholsäure.

Zur Herstellung der wässrigen Mischmicellösung werden vorzugsweise 1 bis 30 g und insbesondere 2 g bis 15 g Gallensäure pro 100 g der gegebenenfalls isotonisierende Zusätze und wasserlösliche Wirkstoffe enthaltenden wässrigen Lösung eingesetzt.

Zur Herstellung der wässrigen Mischmicellösungen können bei dem erfindungsgemäßen Verfahren die gleichen Lipoide verwendet werden, wie bei den vorbekannten Verfahren.

Geeignete Lipoide sind beispielsweise, Monoglyceride, Sulfatide, und insbesondere Phospholipide, wie die Sphingomyeline, die Plasmalogene, die Phosphatidylcholine, die Phosphatidylethanolamine, die Phosphatidylserine, die Phosphatidylinosite und die Cardiolipine auch auch Gemische dieser Lipoide (Dr. Otto-Albert Neumüller: Römpps Chemie-Lexikon; Franck sche Verlagshandlung, Stuttgart(DE) 2665, 3159, 3920 und 4045).

Zur Herstellung der wässrigen Mischmicellösungen werden vorzugsweise 3 bis 40 % und insbesondere 5 bis 20 %. Lipoid pro 100 g der gegebenenfalls isotonisierende Zusätze und/oder wasserlösliche Wirkstoffe enthaltenden wässrigen Lösung verwendet. Das Gewichtsverhältnis zwischen Lipoid und Gallensäure beträgt vorzugsweise 0,1:1 bis 2:1 und insbesondere 0,8:1 bis 2:1.

Als Basen eignen sich zur Herstellung der wässrigen Mischmicellenlösungen nach dem erfindungsgemäßen Verfahren einerseits Alkalihydroxide, wie Lithiumhydroxid, Kaliumhydroxid und insbesondere auch Natriumhydroxid und andererseits organische Stickstoffbasen, sofern sie physiologisch unbedenkliche Salze bilden. Solche Stickstoffbasen sind beispielsweise Ammoniak, primäre, sekundäre oder tertiäre Amine, Ethanolamin, Diethanolamin, Piperazin, Morpholin, Lysin, Ornithin, Arginin, N,N-Dimethylglucamin, das Cholin und insbesondere das N-Methylglucamin und das Trishydroxymethylaminomethan. Diese Basen werden erfindungsgemäß in einer solchen Menge angewendet, daß die Lösungen 0,05 bis 3 Aquivalente Base und insbesondere 0,5 bis 2 Aquivalente Base bezogen auf die Gallensäuren enthalten.

Geeignete wasserlösliche organische Lösungsmittel sind beispielsweise niedere Alkohole, wie Methanol, Ethanol, Propanol oder Isopropanol oder Aceton. Diese Lösungsmittel werden vorzugsweise in einer solchen Menge angewendet, daß die resultierenden Mischungen ebenfalls Lösungen sind. Die genannten Lösungsmittel können sowohl durch Vakuumdestillation als auch durch Umkehrosmose (T.H. Meltzer, Advances in Parenteral Science 13, Filtration in the Pharmaceutical Industry Marcel, Dekker Verlag New York etc., 1. Auflage, 72-74, 483-488 und 838-854; Chem.-Ing. Tech. 61, 1989, 535-544) entfernt werden.

Wenn man das Lösungsmittel durch Lyophylisieren entfernt, ist es zweckmäßig , der Lösung zuvor 20 bis 300 mg eines Mono- oder Disaccharides, wie Glucose oder Galactose oder eines Zuckeralkohols, wie Sorbit oder Mannit zuzusetzen.

Entfernt man das Lösungsmittel durch Ultrafiltration so verwendet man zweckmäßigerweise Filter mit einer Ausschlußgröße von maximal 300000 Dalton

Bei der Umkehrosmose, welche technisch bereits zur Wasseraufbereitung Anwendung findet, wird die zu entfernende Flüssigkeit bekanntlich über eine asymmetrische Membran entfernt, die keine Poren hat. Geeignete Membranen sind beispielsweise solche aus Polydimethylsiloxan oder Polyvinylalkohol von etwa 0,1 bis 2 µm Stärke die auf einer schwammartigen oder gewebsartigen Stützschicht aufgebracht sind. Geeignete Membranmodule sind ebenfalls Kapillar- und Rohrmodule oder auch Plattenmodule oder Spiralwickelmodule. Bezüglich der Entwicklung lösungsmittelselektiver Membranen und ihrer Wirkungsweise sei auf die bereits erwähnte Publikation in der Zeitschrift Chem. Ing. Techn. 60, 1988, 590 ff verwiesen.

Die Umkehrosmose kann nicht nur dazu verwendet werden, solche Lösungsmittel aus wässrigen Dispersionen zu entfernen, die einen höheren Dampfdruck als Wasser haben, sondern eignet sich auch zur Entfernung von Lösungsmitteln mit einem niedrigeren Dampfdruck als Wasser, wie beispielsweise Dimethylformamid, Dimethylsulfoxid oder Acetonitril.

Nach erfolgter Entfernung des Lösungsmittels kann man die erhaltene Mischung gewünschtenfalls mit wässriger Phase verdünnen. Hierbei kann es zweckmäßig sein, eine wässrige Phase zu verwenden, die maximal 10 ml einer neutralisierten Gallensäure und gewünschtenfalls zusätzlich noch isotonisierende Zusätze enthält.

Geeignete in Wasser schwer lösliche oder unlösliche Wirkstoffe sind vorzugsweise solche, deren Löslichkeit in Wasser bei Raumtemperatur 2 % nicht übersteigt. Solche Wirkstoffe sind beispielsweise Pflanzenschutzmittel, wie schwer lösliche Insektizide oder Herbizide und insbesondere schwer lösliche pharmazeutische Wirkstoffe.

In Wasser schwer lösliche oder unlösliche pharmazeutische Wirkstoffe folgender Wirkstoffgruppen eignen sich beispielsweise zur Herstellung der erfindungsgemäßen Arzneimittel:

Gestagen wirksame Steroidhormone wie beispielsweise das 13-Ethyl-17β-hydroxy18,19-dinor-17α-pregn-4-en-20-yl-3-on (=Levonorgestrel), das 13-Ethyl -17β-hydroxy-18,19-dinor-17α-pregna-4,15-dien-20yn-3-on (=Gestoden) oder das 13-Ethyl17β-hydroxy-11-methylen-18,19-dinor-17α-pregn-4-en-20yn (Desorgestrel), Estrogen wirksame Steroidhormone wie 3-Hydroxy-1,3,5(10)estratrien-17-on (=Ostron) oder

1,9-Nor-17α-pregna-1,3,5(10)-trien--20yn-3,17β-diol (Ethinylöstradiol).

Androgen wirksame Steroidhormone wie 17β-Hydroxy-4-androsten-3-on (=Testosteron) und dessen Ester oder 17β-Hydroxy-1α-methyl-5α-androsten-3-on (=Mesterolon).

Antiandrogen wirksame Steroidhormone wie das 17α-Acetoxy-6-chlor-1β,2β-dihydro-3 H-cyclopropa-[1,2]-pregna-1,4,6-trien-3,20-dion (Cypoteronacetat).

Kortikoide wie das 11β,17α,21-Trihydroxy-4-pregnen-3,20-dion (=Hydrocortison), das 11β,17α,21-Trihydroxy-1,4-pregnadien-3,20-dion (=Prednisolon), das 11β,17α-21-Trihydroxy-6α-methyl-1,4-pregnatrien-3,20-dion (=Methylprednisolon) und das 6α-Fluor-11β,21-dihydroxy-16α-methyl-1,4-pregnadien-3,20-dion (=Difluocortolon).

Ergoline wie der 3-(9,10-Dihydro-6-methyl-8α-ergolinyl)-1,1-diethylharnstoff (=Ergolin), der 3-(2-Brom-9,10-dihydro-6-methyl-8α-ergolinyl)-1,1-diethylharnstoff (=Bromergolin) oder der 3-(6-Methyl-8α-ergolinyl)-1,1-diethylharnstoff (=Tergurid).

Antihypertonika wie das 7α-Acetylthio-17α-hydroxy-3-oxo-4-pregnen-21-carbonsäure-γ-lacton (=Spironolacton) oder das 7α-Acetylthio-15β,16β-methylen-3-oxo17α-pregna-1,4-dien-21,17-carbolacton (=Mespirenon).

Antikoagulantia wie die 5-[Hexahydro-5-hydroxy-4-(3-hydroxy-4-methyl-1-octen-6-ynyl)-2(1H)-pentalenyliden)]-pentansäure (=Iloprost).

Psychopharmaka wie das 4-(3-Cyclopentyloxy-4-methoxy-phenyl-2-pyrrolidon (=Rolipram) und das 7-Chlor-1,3-dihydro-1-methyl-5-phenyl-2H-1,4-benzodiazepin-2-on (=Diazepam).

Carotinoide wie das α-Carotin und das β-Carotin.

Fettlösliche Vitamine, wie Vitamine der Vitamin A-, Vitamin D-, Vitamin E- und Vitamin K-Gruppe.

Eine besonders bevorzugte Gruppe sind β-Carboline, wie sie beispielsweise in den Europäischen Patentanmeldungen 234,173 und 239,667 beschrieben sind. Als β-Carboline seien beispielsweise genannte der 6-Benzoyloxy-4-methoxymethyl-βcarbolin-3-carbonsäure-isopropylester (=Becarnil) und der 5-(4-Chlorphenoxy)4-methoxymethyl-β-carbolin-3-carbonsäure-isopropylester (=Cl-Phocip).

Die nach dem erfindungsgemäßen Verfahren hergestellten wässrigen Mischmicelllösungen können gewünschtenfalls isotonische Zusätze enthalten, um deren osmotischen Druck zu erhöhen. Geeignete Zusätze sind beispielsweise anorganische oder organische Salze oder Puffersubstanzen, wie Natriumchlorid, Phosphat-Puffer, Citrat-Puffer, Glycin-Puffer, Citrat-Phosphat-Puffer, TRIS-HCl-Puffer, Maleat-Puffer, etc. Mono- oder Disaccharide, wie Glucose, Lactose, Saccharose, Zuckeralkohole, wie Mannit, Sorbit, Xylit oder Glycerin oder wasserlösliche Polymere, wie Dextran oder Polyethylenglykol.

Diese isotonisierenden Substanzen werden üblicherweise in solchen Konzentrationen zugesetzt, daß die entstehende wässrige Mischmicellösung einen osmotischen Druck von 5 - 1000 mosm - bei Injektionslösungen optimalerweise 300 mosm - aufweist.

Ferner können die wässrigen Mischmicellösungen noch zusätzliche wasserlösliche Wirkstoffe enthalten, um Kombinationspräparate herzustellen. Beispiele solcher Kombinationspräparate sind Mischungen aus wasserlöslichen und fettlöslichen Vitaminen oder Präparate die neben Kortikoiden noch wasserlösliche Antibiotika enthalten.

Die Herstellung der wasserlöslichen Mischmicellösungen erfolgt - abgesehen von den im Patentanspruch 1 aufgeführten Bedingungen mittels konventioneller Methoden, indem man die Mischungen unter kräftigem Rühren auf die im Patentanspruch 1 aufgeführten Temperaturen erhitzt.

Da die Lipoide und auch einige Wirkstoffe oxidationsempfindlich sind, wird das Verfahren zweckmäßigerweise unter einer Inertgasatmosphäre, wie Stickstoff oder Argon durchgeführt und die erhaltenen wässrigen Mischmicellösungen durch Zugabe von Antioxidantien, wie Natriumascorbat, Tocopherol oder Natriumhydrogensulfit stabilisiert.

Das erfindungsgemäße Verfahren hat den Vorzug, daß es in technischem Maßstab wesentlich einfacher durchführbar ist als die vorbekannten Verfahren.

Die zu seiner Durchführung erforderlichen Verfahrensschritte Lösen, Mischen und Vakuumdestillation oder Umkehrosmose, sind technisch kontinuierlich durchführbar und wenig aufwendig. Zudem hat das erfindungsgemäße Verfahren den Vorzug, daß die thermische Belastung der Komponenten geringer ist als bei den vorbekannten Verfahren, dies insbesondere dann, wenn man zur Entfernung der Lösungsmittel das Verfahren der Umkehrosmose anwendet. Nach erfolgter Herstellung kann die erhaltene wässrige Mischmicellösung sterilfiltriert und/oder bei 100° C bis 140° C hitzesterilisiert werden.

Die nachfolgenden Ausführungsbeispiele dienen zur näheren Erläuterung des erfindungsgemäßen Verfahrens.

## Beispiel 1

400 ml einer wässrigen Lösung, die 2,138 Natriumhydroxid enthält, werden in einem 2 1 Rundkolben vorgelegt. Dann versetzt man sie mit 100 ml einer ethanolischen Lösung, die 45 g Phospholipid ((Phospholipon 100 L, Hersteller A. Nattermann & Cie., DE-5000 Köln) und 27,1 g Glykocholsäure enthält. Es entsteht eine klare Mischung leicht mit gelblicher Färbung und einem ph-Wert von 6,5.

Man entfernt das Ethanol durch Umkehrosmose, indem man die Lösung in einer Apparatur für die Umkehrosmose (Membra-Fil P-28. Fa. Buchi, Göppingen, Membran DRC-1000) von 500 ml auf 250 ml aufkonzentriert. Der Druck beträgt 35 bar. In einem zweiten Schritt wird das Konzentrat mit einer 3,5 mM Lösung von neutralisierter Glykocholsäure auf das Ausgangsvolumen verdünnt. Die erhaltene Mischmizellformulierung enthält danach weniger als 0,01 mg/ml Ethanol.

Die Zusammensetzung ist wie folgt:

| | | |
|---|---|---|
| Phospholipon: Hersteller Nattermann AG, DE-5000 Köln | | 90,0 mg |
| Glykocholsäure: | | 52,1 mg |
| Natriumhydroxid: | | 4,3 mg |
| Wasser | ad | 1,0 ml |
| pH-Wert: | | 6,6 |

Beispiel 2

800 ml einer wässrigen Lösung mit 2,8 g Kaliumhydroxid werden mit 120 ml einer ethanolischen Lösung versetzt, die 45 g Phospholipon und 27 g Glykocholsäure enthält. Es entsteht eine klare Lösung, deren pH-Wert auf 6,5 mit 0,1 N Kaliumhydroxidlösung eingestellt wird. Zu dieser Lösung werden 18 g Sorbit gegeben und gerührt bis zur vollständigen Auflösung.

Die ethanolhaltige Mischmizellösung wird in eine Ultrafiltrationsanlage (Amicon GmbH, DE 8510 Witten; Typ DC 2, Membran: HIP 30-20) überführt. Bei einem Membrandifferenzdruck von maximal 1 bar wird ultrafiltriert. Das Volumen des entfernten Ultrafiltrats wird kontinuierlich durch eine 10 mM neutralisierte Glykocholsäurelösung ersetzt. Beträgt das Ultrafiltratvolumen 2,5 1, wird die kontinuierliche Zuführung unterbrochen und die Mischmizellösung von 1 l auf 500 ml aufkonzentriert.

Die Formulierung hat folgende Zusammensetzung:

| | | |
|---|---|---|
| Phospholipon: | | 88,9 mg |
| Glykocholsäure | | 58,0 mg |
| Kaliumhydroxid: | | 6,4 mg |
| Wasser | ad | 1,0 ml |
| pH-Wert: | | 6,8 |

Beispiel 3

Unter den Bedingungen des Beispiels 2 wird eine Mischmizellösung hergestellt, jedoch mit dem Unterschied, daß die wäßrige Lösung zusätzlich 50 mg Natrium EDTA und die ethanolische Lösung zusätzlich 580 mg Wirkstoff (Cl-Phocip) enthält.

Die erhaltene Mischmizellformulierung hat folgende Zusammensetzung:

| | | |
|---|---|---|
| Phospholipon: | | 89,2 mg |
| Glykocholsäure: | | 52,3 mg |
| Kaliumhydroxid: | | 6,1 mg |
| Cl-Phocip: | | 1,01 mg |
| Wasser | ad | 1,0 ml |
| pH-Wert: | | 6,7 |

**Patentansprüche**

1. Verfahren zur Herstellung wässriger Mischmicellösungen, enthaltend aus Lipiden und Salzen von Gallensäuren gebildete Mischmicellen, in denen gewünschtenfalls in Wasser schwer lösliche oder unlösliche Wirkstoffe solubilisiert sind, dadurch gekennzeichnet, daß man Mischungen aus
   a) Lösungen, welche die Lipide, die freien Gallensäuren und gegebenenfalls die in Wasser schwer löslichen oder unlöslichen Wirkstoffe in einem wasserlöslichen organischem Lösungsmittel enthalten und

b) Lösungen die bezogen auf die Gallensäuren 0,05 bis 3 Aquivalente Basen und gegebenenfalls isotonisierende Zusätze und/oder wasserlösliche Wirkstoffe enthalten bereitet, das organische Lösungsmittel durch Ultrafiltration, Lyophylisieren, Vakuumdestillation oder Umkehrosmose entfernt und die erhaltene Mischung gewünschtenfalls mit wässriger Phase verdünnt.

2. Verfahren zur Herstellung von Mischmicellösungen gemäß Patentanspruch 1, dadurch gekennzeichnet, daß man die Lösungen a) in derartig verdünnter Form anwendet, daß die resultierenden Mischungen ebenfalls Lösungen sind.

3. Verfahren zur Herstellung wässriger Mischmicellösungen gemäß Patentanspruch 1 und 2, dadurch gekennzeichnet, daß man als Gallensäure ein 5β-Cholan-24-säure-Derivat der allgemeinen Formel

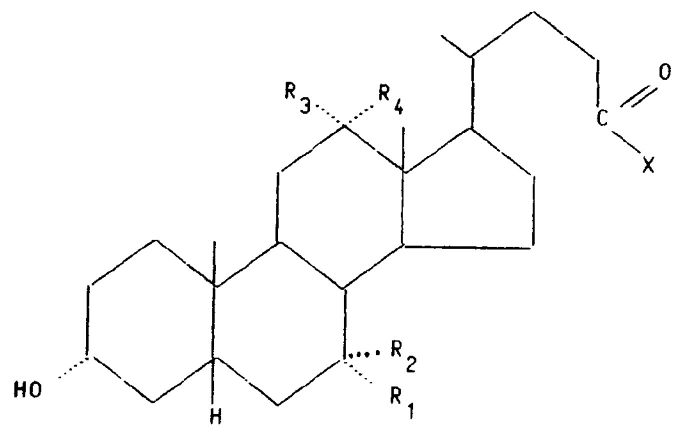

worin
$R_1$ und $R_2$ sowie $R_3$ und $R_4$ gemeinsam eine Oxogruppe, zwei Wasserstoffatome oder ein Wasserstoffatome und eine Hydroxygruppe bedeuten und X eine Hydroxygruppe oder eine Gruppierung der Formel $-NH-CH_2-CO_2H$ oder $-NH-(CH_2)_2-SO_3H$ darstellt, verwendet.

4. Verfahren zur Herstellung wässriger Mischmicellösungen gemäß Patentanspruch 1 bis 3, dadurch gekennzeichnet, daß man als Lipide Phospholipide verwendet.

5. Verfahren zur Herstellung wässriger Mischmicellösungen gemäß Patentanspruch 1-4, dadurch gekennzeichnet, daß man als Basen Natriumhydroxid oder Kaliumhydroxid verwendet.

6. Verfahren zur Herstellung wässriger Mischmicellösungen gemäß Patentanspruch 1 bis 5, dadurch gekennzeichnet, daß man als in Wasser schwer lösliche oder unlösliche Wirkstoffe, in Wasser schwer lösliche oder unlösliche pharmazeutische Wirkstoffe verwendet.

7. Verwendung von gemäß Patentanspruch 6 hergestellten wässrigen Mischmicellösungen zur Herstellung von Injektionslösungen.

## Claims

1. Process for the manufacture of mixed micelle solutions containing mixed micelles formed from lipids and salts of bile acids, in which, if desired, active ingredients that are sparingly soluble or insoluble in water have been solubilised, characterised in that

a) solutions that contain, in a water-soluble organic solvent, the lipids, the free bile acids and, optionally, the active ingredients that are sparingly soluble or insoluble in water and

b) solutions that contain, based on the bile acids, from 0.05 to 3 equivalents of bases and, optionally, isotonising additives and/or water-soluble active ingredients are prepared,

the organic solvent is removed by ultrafiltration, lyophilisation, vacuum distillation or reverse osmosis, and, if desired, the resulting mixture is diluted with aqueous phase.

2. Process for the manufacture of mixed micelle solutions according to patent claim 1, characterised in that the solutions a) are used in such a dilute form that the resulting mixtures are also solutions.

3. Process for the manufacture of aqueous mixed micelle solutions according to patent claims 1 and 2, characterised in that there is used as bile acid a 5β-cholane-24-acid derivative of the general formula

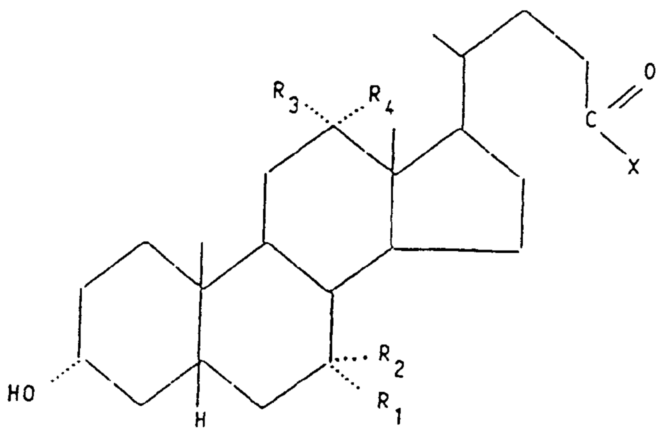

wherein
$R_1$ and $R_2$ and also $R_3$ and $R_4$ together represent an oxo group, two hydrogen atoms or one hydrogen atom and one hydroxy group and
X represents a hydroxy group or a grouping of the formula $-NH-CH_2-CO_2H$ or $-NH-(CH_2)_2-SO_3H$.

4. Process for the manufacture of aqueous mixed micelle solutions according to patent claims 1 to 3, characterised in that phospholipids are used as lipids.

5. Process for the manufacture of aqueous mixed micelle solutions according to patent claims 1 to 4, characterised in that sodium hydroxide or potassium hydroxide is used as base.

6. Process for the manufacture of aqueous mixed micelle solutions according to patent claims 1 to 5, characterised in that pharmaceutical active ingredients that are sparingly soluble or insoluble in water are used as active ingredients that are sparingly soluble or insoluble in water.

7. Use of aqueous mixed micelle solutions manufactured according to patent claim 6 for the manufacture of injection solutions.

## Revendications

1. Procédé de préparation de solutions aqueuses de micelles mixtes comprenant des micelles mixtes formées de lipides et de sels d'acides biliaires, dans lesquelles sont solubilisées si l'on veut des matières actives très peu solubles ou insolubles dans l'eau, procédé caractérisé en ce que l'on prépare des mélanges :
   a) de solutions contenant dans un solvant organique hydrosoluble les lipides, les acides biliaires libres et le cas échéant les matières actives peu solubles ou insolubles dans l'eau, et
   b) de solutions contenant par rapport aux acides biliaires de 0,05 à 3 équivalents de bases et le cas échéant des additifs isotonisants et/ou des matières actives hydrosolubles,
   on élimine ensuite le solvant organique par ultrafiltration, lyophilisation, distillation sous vide ou osmose inverse puis, si on le souhaite, on dilue le mélange ainsi obtenu avec une phase aqueuse.

2. Procédé selon la revendication 1, caractérisé en ce que les solutions a) sont dans un état dilué tel que les mélanges obtenus sont également des solutions.

3. Procédé selon les revendications 1 et 2, caractérisé en ce que l'acide biliaire est un dérivé de l'acide 5-β-cholanique-24 de formule générale

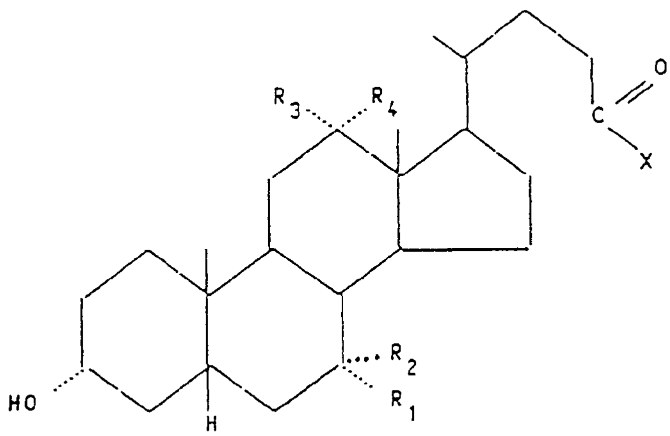

dans laquelle
$R_1$ et $R_2$ ainsi que $R_3$ et $R_4$ représentent ensemble un groupe oxo, ou bien deux atomes d'hydrogène ou encore un atome d'hydrogène et un groupe hydroxylique, et X désigne un groupe hydroxylique ou un groupe $-NH-CH_2-CO_2H$ ou $-NH-(CH_2)_2-SO_3H$.

4. Procédé selon l'une des revendications 1 à 3, caractérisé en ce que l'on utilise comme lipides des phospholipides.

5. Procédé selon l'une des revendications 1 à 4, caractérisé en ce que la base est de l'hydroxyde de sodium ou de potassium.

6. Procédé selon l'une des revendications 1 à 5, caractérisé en ce que l'on ajoute comme matières actives peu solubles ou insolubles dans l'eau des produits pharmaceutiques peu solubles ou insolubles dans l'eau.

7. L'emploi de solutions aqueuses de micelles mixtes obtenues selon la revendication 6 pour en préparer des solutions injectables.